# EUROPEAN PATENT APPLICATION

(11) **EP 1 467 209 A1**
(43) Date of publication of application: **13.10.2004**
(21) Application number: 03008457.8
(22) Date of filing: 11.04.2003
(51) Int. Cl.: G01N 33/543

(54) **Solid phase screening method**

(71) Applicant: Graffinity Pharmaceuticals Aktiengesellschaft, 69120 Heidelberg (DE)
(72) Inventor: Schollmeier, Klaus, Dr., 69502 Hemsbach (DE); Vanier, Cécile, Dr., 69221 Dossenheim (DE); Ottleben, Holger, Dr., 69155 Heidelberg (DE); Maier, Sabine, Dr., 69124 Heidelberg (DE)
(74) Representative: Büchel, Edwin, Dr.

(57) **Abstract**

The invention relates to screening methods for the identification of ligands and/or ligand precursors which may interact with a target, whereby the ligands are synthesized on a solid phase in the presence of said target.

## Description

The invention relates to screening methods for the identification of ligands and/or ligand precursors which may interact with a target, whereby the ligands are synthesized on a solid phase in the presence of said target.

It is generally accepted that the drug discovery process involves the analysis of a multitude of chemical compounds in order to identify a potential drug candidate. For this purpose, biomolecular interactions of the chemical compounds are often studied via target-ligand systems, the target typically being a biomacromolecule (e. g. a protein) and the ligand being a "probe", i. e. usually a low molecular weight molecule (peptide, oligonucleotide, or so-called small organic molecule). Such ligands exhibit specific structural features which may interact with the target if the latter possesses corresponding structural elements.

In order to analyze the hundreds or thousands of compounds comprising a compound library, screening assays have to be adapted for high-throughput-screening (HTS) which is usually based on microplate systems and robotic liquid handling technology. However, conventional HTS methods can usually be applied only if the target has been validated and functionally characterized. Often, a ligand or substrate has to be known for the target.

HTS can be performed in solution or on solid phase. The main advantage of solid phase screening is the inherent potential towards miniaturization and automation of the assay equipment. For solid phase screening, either the predominantly macromolecular target or the candidate target binding molecule, i. e. the ligand can be immobilized. While the first alternative is already in use in order to detect binding partners, the second alternative of immobilizing the ligand is considered to be unsuitable for screening because of the putative steric hindrance of the interaction target (see Gordon E. M. and Kerwin J. F., Combinatorial Chemistry and Molecular Diversity in Drug Discovery, Wiley-Liss 1998, p. 424-425).

Besides finding suitable solid phase screening methods, the question of how this multitude of compounds (ligands) to be screened can best be obtained has to be solved.

Solid phase synthesis, especially solid phase combinatorial chemistry offers the best tools for the synthesis of libraries of ligands. The advantage of solid phase synthesis and combinatorial chemistry, particularly the efficiency of automated parallel synthesis, lies in its ability to produce hundreds and thousands of compounds on a very short time frame.

Methods for the screening on a solid support that are suitable for HTS have been published.

G. McBeath and S. L. Schreiber (Science 2000,289: 1760-63; G. McBeath et al., J. Am. Chem. Soc. 1999,121: 7967-68) use a high precision robot designed to manufacture DNA microarrays to spot droplets of thiol-containing small molecules onto maleimide-derivatized glass slides at high spatial densities (1600 spots per cm²). Each slide can then be probed with a differently tagged protein and binding events are detected by a fluorescence-linked assay.

P. J. Hergenrother et al. (J. Am. Chem. Soc. 2000,122: 7849-50) use the same method to immobilize alcohol-containing small molecules on glass slides. This array is capable of detecting known ligands from a compound library containing 80 compounds.

Scharn et al. (J. Comb. Chem. 2000,2 : 361-369) describe a method for parallel synthesis and screening of membrane-bound small organic molecules such as 1,3,5-triazines. A microarray created by this method can contain up to 8 000 samples. Ligand-target interactions are detected via an enzyme-linked assay.

All these screening methods are performed on a solid phase and benefit from the use of the same solid phase for synthesis purposes but none of these methods accomplish the synthesis in the presence of the target in order to shorten the time for identifying high potential ligands which can interact with a target.

The object of the invention is to provide improved screening methods for the identification of ligands or ligand precursors which may interact with a target, whereby the ligands are synthesized on a solid phase in the presence of said target so that screening and solid phase syntheses are combined to accelerate the identification of ligands and/or ligand precursors and/or where the target of interest may trigger or promote the formation of ligands having increased binding properties.

Thus, the method of the present invention comprises the steps:
(a) providing a set of ligand precursors immobilized on a solid phase having at least one functional group capable of covalently binding reactants;
(b) adding the at least one reactant to the set of immobilized ligand precursors;
(c) adding the target to the solid phase using conditions which are suitable for the target and said conditions allow the reaction of the ligand precursors with the reactant(s) via said functional group(s) to form ligands, whereby the adding of the target occurs before step (b), simultaneously with the adding of the set of reactants or after step (b) but before the reaction of forming the ligands is completed;
(d) detecting a binding value or change in the binding value of the interaction between the target and each ligand or ligand precursor;
(e) identifying at least one ligand precursor causing an increased binding value detected in step (d).

The object of the invention is also achieved by a method comprising the steps:
(a) providing a set of ligand precursors immobilized on a solid phase having at least one functional group capable of covalently binding reactants;
(b) adding the target to the set of ligand precursors;
(c) determining a binding value of the ligand precursors / target interaction;
(d) adding at least one reactant to the set of immobilized ligand precursors and the target;
(e) subjecting the mixture to conditions allowing a reaction of the ligand precursors with the reactant(s) via said functional group(s) to form ligands;
(f) determining a binding value of the respective ligand / target interaction;
(g) identifying at least one ligand precursor causing an increased binding value from step (f) compared to step (c).

In one embodiment of the invention there is exactly one reactant which is added. This is advantageous for the identification of the ligand. Once, having identified the ligand precursor interacting with the target, it is easy to determine the ligand structure formed by the reaction between the ligand precursor and the reactant. Therefor, an additional step to identify the ligand is not necessary.

In general terms, ligand precursor means any chemical molecule that can react with a second chemical molecule (reactant) to form a ligand, e.g. a fragment of the ligand that can be chemically combined covalently with the reactant subject to an addition or substitution or other chemical reaction via at least one functional group. Ligand precursors form the set of reactants that is immobilized on the solid phase. Preferably, at least 10 more preferred at least 96 different ligand fragments are used in the screening method.

Ligand fragments as well as reactants may be fragments of known binders to the target.

The term "ligand" means any molecule of chemical or biological nature having functionalities or structural motifs which may lead to a binding interaction of a target under suitable conditions. Preferably, the molecular weight of the ligands is less than 1000 g/mol, more preferred less than 750 g/mol, most preferred less than 500 g/mol.

"Target" means any molecule, preferably biomolecule, with a molecular weight typically greater than 500 Da, preferably greater than 1000 Da. Examples are DNA, RNA, oligonucleotides, proteins, like membrane proteins, hormones, receptor proteins, transport proteins, channel proteins, antibodies or enzymes, and analogs, but also synthetic molecules, such as fusion proteins or synthesized primers. Preferably, the target is a protein.

In another embodiment a plurality of reactants, preferably at least a set of 4 reactants, more preferred at least 10 reactants, most preferred at least 100 reactants, is added. The addition can be realized subsequently or simultaneously. In a preferred embodiment the set of reactants is added as a solution comprising a mixture of all reactants.

In such a case the identification of ligands is not automatically given by the determination of the ligand precursor. Preferably, an additional step identifying the ligand formed by the ligand precursor causing the increased binding value follows after identification of the ligand precursor. One way to find out what kind of ligand is formed is to characterize the ligand directly with or without conjugation with the target. Another way is to select the specific reactant set member causing the increased binding value so that the ligand is identified by the reacting parts as outlined before. The identification of a specific reactant within the whole set of reactant can be accomplished by repeating the screening methods and subsequently reducing the number of the reactants until the increased binding signal disappears.

For the identification of the reactant, ligand respectively, within a set or mixture of a multitude of reactants it may be necessary to remove unbound reactants from the solid phase, e.g. by a washing step.

For the identification of the ligand it may be necessary to remove the ligand from the solid phase and identify the structure subsequently. An appropriate method for removal and analysis is the so called MALDI mass spectrometry (MALDI MS).

It is crucial that the reaction conditions which allow the covalent reaction of the ligand precursors with the reactant(s) are selected so that these conditions are suitable for the target to conserve its binding properties, preferably its activity. Preferably, physiological condition are used, e.g. neutral pH, moderate temperature, buffer solutions or aqueous solutions optionally containing other solvents, e.g. DMSO.

The covalent binding of ligand precursor and reactant may be reversible or irreversible. Reversible binding occurs when the half time of the formation and cleavage of the product is typically less than one month, preferably less than one day and more preferred less than an hour. Irreversible binding is any covalent binding that is not reversible.

For the purpose of "target driven" formation of potential ligands, it is essential that the target is added to the reaction mixture before the reaction of forming the ligands is completed. For an irreversible reaction formation is completed when no additional product is formed. Within the meaning of the invention the forming of a ligand in a reversible reaction is never completed, even in the state of equilibrium, because the target may influence the formation of the ligand (product) and decomposition by changing the conditions of equilibrium.

Examples for ligand precursors and/or reactants for reversible binding are independently amines, amidines, guanidines, hydrazines and hydrazides, thiosemicarbazides, hydroxylamines, thiols, alcohols and phenols, hydroperoxides, boronic acids, carbonyl compounds (especially aldehydes, ketones, carboxylic acids, esters, carbamates, ureas), thio-carbonyl compounds and di-(thio) carbonyl compounds, sulfoxides, sulfones, sulfinic acids, sulfonic acids, olefines, alkynes.

Examples for ligand precursors and/or reactants for irreversible binding are independently amines, hydrazines and hydrazides, thiosemicarbazides, hydroxylamines, thiols, alcohols and phenols, hydroperoxides, boronic acids, carbonyl compounds (especially aldehydes, ketones, carboxylic acids, esters, carbamates, ureas), thio-carbonyl compounds and di-(thio) carbonyl compounds, sulfoxides, sulfones, sulfinic acids, sulfonic acids, olefines, alkynes, halides (especially arylic or benzylic), halo-ketones, isocyanates and isothiocyanates, azides, nitriles, phosphorous compounds (especially phosphonates esters, phosphonic acids), nitroso compounds.

In a preferred embodiment the connection of the ligand fragment with the reactant is given by the formation of a new covalent bond (single, double bond) involving a chemical addition or substitution reaction.

Examples of reversible bond formations or reactions are, e.g. Imine formations, Oxime formations, hydrazone and acyl-hydrazone formations, thiosemicarbazone formations, amide formations, acetal formations, thiazolidines or oxazolidine formations, ester or thioester formations, disulfide formations, borate esters formations, aldol reactions, Diels-Alder reactions, Michael additions, Alkene metathesis, Hydroxamic acid formations.

Preferably, examples for reversible reactions or bond formations are those where carbonyl groups react under the formation of imines, amides, (thio)acetals, (thio)esters, (thio)semicarbazones, thiazolidines or oxazolidines, Hydroxamic acids.
In particular, the reaction of amino groups and derivatives thereof with carbonyl groups to imines, oximes or hydrazones is well adapted.
Further useful examples are the formation of disulfides and acetals as well as the alcohol exchange in esters and borate esters. Reactions such as Michael reactions, aldol reactions, reversible Diels-Alder and other thermal or photo-induced rearrangements or all kinds of metathesis using catalysts that may be soluble in aqueous solutions are further examples.

To a certain extend, all reversible reactions can be driven to irreversible reactions by changing the reaction conditions.

Examples for the irreversible bond formation or reactions are imine formations, oxime formations, hydrazone and acyl-hydrazone formations, thiosemicarbazone formations, amide formations, carbamate formations, acetal formations, ether formations, urea or thiourea formations, ester or thioesters formations, disulfide formations, borate esters formations, aldol reactions, Diels-Alder reactions, Michael's additions, alkene metathesis and all kinds of metal-catalyzed coupling reactions, heterocycles formations, condensation reactions, multicomponent reactions.

Well suited examples for irreversible reactions or bond formations are those where amino groups and derivatives thereof react with electrophiles such as (thio)-isocyantes, carbamates or halides under the formation of (thio)-ureas, or amines.
In particular, heterocycle formations whereby carbonyl compounds and derivatives thereof, bi-carbonyl compounds (or synthetic equivalents) react with amino groups (and derivatives thereof) to lead to the formation of pyrroles, pyridines, pyrrazoles, isoxazoles is well adapted.
Further examples of such reactions are all C-C, C-N, or C-O bond formations under couplings using catalysts that may be soluble in aqueous solutions (especially Suzuki, Heck, Buchwald or Mitsunobu-type couplings).

In another embodiment the ligand precursors have two different functional groups capable of binding two different sets of reactants.

Within the meaning of the present invention functional groups are not necessarily restricted to small functionalities, like amine, hydroxyl, carboxyl etc., but includes also more complex systems like Michael acceptors, or [2+4] cyclo addition systems or the like.

Having "different functional groups" means that it is possible to address in a first reaction step one reaction site and in a second step a different reaction site is targeted. This may be achieved by using different protective groups for the same functional group, e.g. amine, laying within the scope of the present invention, too. Consequently, in such a case the set of different reactants may consists of only one chemical class, e.g. carbonic acids.

Examples for functional groups which are suitable as "different functional group" are amino groups, hydrazines and hydrazides, thiosemicarbazides, hydroxylamines, thiol groups, alcohol and phenol groups, borates derivatives, carboxy groups and derivatives thereof, thio-carboxy and di-(thio) carboxy groups and derivatives thereof, sulfo groups, sulfonato groups and sulfamoyl groups, olefines, alkynes, halides (especially arylic or benzylic), isocyanate and isothiocyanate groups, nitriles groups.

In a preferred embodiment the ligand precursors are immobilized on a solid phase comprising an organic layer attached to a metal film on a solid support.

Preferably, the support used to immobilize the ligand precursors a noble metal (silver, palladium, platinum ; especially gold) or a substrate the surface of which is at least partly covered with a layer of such a metal.

Particularly preferred are gold surfaces. The material used depends on the detection method for the determination of the binding value.

The term "binding value" means a measured value of the signal derived from the detection system and relates functionally to the binding force of the interaction pair ligand precursor/target or ligand/target.

If reflection-optical methods, such as surface plasmon resonance (SPR) are used, the preferred solid supports comprise glass or a light transmitting polymer coated with a thin gold film.

In such a case, the binding value corresponds to the SPR signal, achieved by the SPR detection system. In appropriate SPR system is disclosed in WO-A-01/63256.

To identify a ligand precursor or ligand binding values are detected resulting from corresponding signals. There are several possibilities for such an identification.
In a first example, only the highest signals are used. A comparison with other signals is not necessary. In a second example, immediately after addition of the reactant(s) a signal is detected and after equilibration a second signal is detected and both are compared, i.e. the change is detected and form the basis for the identification of the ligand precursor(s) or ligands. In a third example the signals after equilibration are compared with the signals obtained during the interaction of the ligand precursors with the target. Preferably, both measurement are made using the identical solid phase set up, but alternatively, earlier measurements can be used. It deems obvious for a person skilled in the art to find alternatives for the identification of ligands or ligand precursors analyzing the binding values.

Preferably the ligand precursors are position addressable so that these are identifiable by the coordinates on the solid phase.

More preferably, the immobilized ligand precursors are arranged in a two-dimensional array formate, i. e. on a microarray comprising discrete fields the spatial location of which can be easily identified and addressed. Each location of the array carries one type of ligand precursor from a known source and with a known structure. Suitable microarrays for the purpose of the present invention include, e. g., a two-dimensional planar solid support with a plurality of position-addressable reaction areas for the immobilization of samples of small size, preferably in a regular pattern for screening purposes. As regards the number of reaction areas, conventional microplates can be used, such as those of the 96-well or 384-well type.

preferably, if a microarray is used as a solid support, the number of different ligand fragments corresponds to the number of reaction areas in the array.

In a preferred embodiment, the organic layer comprises a self assembled monolayer (SAM) comprising anchor molecules and optionally dilution molecules.

Using such a system, ligands can be removed by photo cleavage of the anchor molecules. Another advantage is, that the screening methods can be carried out using different dilutions to adopt an optimal signal range.

The ligand precursors are preferably immobilized on the support via anchor molecules comprising at least two functional moieties at opposite ends of the anchor, one being able to bind with the surface of the support, the other one to bind the ligand precursor. Such anchors should be able to form a self-assembling monolayer (SAM) on the surface of the support. Suitable anchor structures are, e. g., disclosed in WO-A-00/73796 and WO-A-01/92883, and those are preferred for the purpose of the present invention which carry a thiol functionality to interact with the solid support. Suitable structural elements that support SAM formation and, at the same time, allow the adjustment of suitable distances between the support and the ligand, are described in WO-A-00/73796. The above documents also provide a detailed description of methods for the synthesis of such anchors and of suitable binding matrices containing them together with ligand precursors (in these documents called ligands) attached to them.

The ligand precursor may be bound, preferably covalently, with the anchor structure prior to its immobilization on the support. In this case, complete ligand precursor-anchor-conjugates (LAC) are contacted with and bound to the support as disclosed in WO-A-00/73796.

However, for the present method, the strategy disclosed in WO-A-01/92883, where the anchor molecules are immobilized on the support in an activated form and are subsequently bound with the ligand precursor, has proven to be particularly advantageous. In this latter approach, anchor structures are synthesized so as to carry a reactive "head group", i. e. a group which allows a selective and preferably quantitative reaction of the thus activated anchor with the ligand precursor. It should be understood that this head group is at a terminal of the anchor structure facing away from the support on which the anchor is immobilized. Depending on the chemical nature of the head group, this strategy may require a chemical modification of the ligand precursor so as to carry a specific functionality which is able to react with the head group of the activated anchor. Once the activated anchors are immobilized on the solid support, they can be reacted with the ligand precursor / modified ligand precursor in a separate step to provide the binding matrix.

Usually this reaction is conducted with an excess of the ligand precursor / modified ligand precursor to get a preferably quantitative conversion of the reactive "head groups" of the anchor molecules. An advantage of this method is that the ligand precursor concentration on the surface is solely determined by the concentration of anchor molecules and not by the concentration of ligand precursors in the added solution. This is of particular advantage if many ligand precursors have to be analyzed in parallel. Therefore, the reproducibility and the comparability of different measurements can be improved. Mercaptophilic head groups as listed in WO-A-01/92883 which covalently bind the ligand precursor are preferred for this purpose. Among them, the method of providing a binding matrix by reacting a thiol-containing ligand precursor with immobilized anchors carrying a maleimide as a head group has been proven particularly advantageous. In this case, a thiol functionality is introduced into the ligand precursor to be screened during or after their synthesis. Once the surface of the support is covered with the anchor structures, the thiol-functionalized ligand precursors are reacted with the mercaptophilic head group to provide ligand precursor anchor conjugates immobilized on the support. A preferred alternative to introduce the thiol functionality is the reaction of the ligand precursor with a spacer molecule containing a thiol group. Suitable spacer molecules are disclosed in WO-A-02/063299.

## Claims

1. A screening method for the identification of ligands and/or ligand precursors which may interact with a target, whereby the ligands are synthesized on a solid phase in the presence of said target, which method comprises the steps:
(a) providing a set of ligand precursors immobilized on a solid phase having at least one functional group capable of covalently binding reactants;
(b) adding the at least one reactant to the set of immobilized ligand precursors;
(c) adding the target to the solid phase using conditions which are suitable for the target and said conditions allow the reaction of the ligand precursors with the reactant(s) via said functional group(s) to form ligands, whereby the adding of the target occurs before step (b), simultaneously with the adding of the set of reactants or after step (b) but before the reaction of forming the ligands is completed;
(d) detecting a binding value or change in the binding value of the interaction between the target and each ligand or ligand precursor;
(e) identifying at least one ligand precursor causing an increased binding value detected in step (d).

2. A screening method for the identification of ligands and/or ligand precursors which may interact with a target, which method comprises the steps:
(a) providing a set of ligand precursors immobilized on a solid phase having at least one functional group capable of covalently binding reactants;
(b) adding the target to the set of ligand precursors;
(c) determining a binding value of the ligand precursors / target interaction;
(d) adding at least one reactant to the set of immobilized ligand precursors and the target;
(e) subjecting the mixture to conditions allowing a reaction of the ligand precursors with the reactant(s) via said functional group(s) to form ligands;
(f) determining a binding value of the respective ligand / target interaction;
(g) identifying at least one ligand precursor causing an increased binding value from step (f) compared to step (c).

3. The method according to claim 1 or 2, wherein one reactant is added.

4. The method according to claim 1 to 2, wherein a plurality of reactants, preferably at least a set of 4 reactants, is added.

5. The method according to claim 4, comprising an additional step (f), (h) respectively, identifying the ligand formed by the ligand precursor causing the increased binding value.

6. The method according to any of the claims 1 to 5, wherein the covalently binding is irreversible.

7. The method according to any of the claims 1 to 5, wherein the covalently binding is reversible.

8. The method according to claim 6, wherein the irreversible bond formation is selected from the group consisting of imine formations, oxime formations, hydrazone and acyl-hydrazone formations, thiosemicarbazone formations, amide formations, carbamate formations, acetal formations, ether formations, urea or thiourea formations, ester or thioesters formations, disulfide formations, borate esters formations, aldol reactions, Diels-Alder reactions, Michael's additions, alkene metathesis and all kinds of metal-catalysed coupling reactions, heterocycles formations, condensation reactions and multicomponent reactions.

9. The method according to claim 7, wherein the reversible bond formation is selected from the group consisting of Imine formations, Oxime formations, hydrazone and acyl-hydrazone formations, thiosemicarbazone formations, amide formations, acetal formations, thiazolidines or oxazolidine formations, ester or thioester formations, disulfide formations, borate esters formations, aldol reactions, Diels-Alder reactions, Michael additions, Alkene metathesis and Hydroxamic acid formations.

10. The method according to claim 6, wherein the ligand precursor and/or reactant is independently selected from the group consisting of amines, hydrazines and hydrazides, thiosemicarbazides, hydroxylamines, thiols, alcohols and phenols, hydroperoxides, boronic acids, carbonyl compounds (especially aldehydes, ketones, carboxylic acids, esters, carbamates, ureas), thio-carbonyl compounds and di-(thio) carbonyl compounds, sulfoxides, sulfones, sulfinic acids, sulfonic acids, olefines, alkynes, halides (especially arylic or benzylic), halo-ketones, isocyanates and isothiocyanates, azides, nitriles, phosphorous compounds (especially phosphonates esters, phosphonic acids) and nitroso compounds.

11. The method according to claim 7, wherein the ligand precursor and/or reactant is independently selected from the group consisting of amines, amidines, guanidines, hydrazines and hydrazides, thiosemicarbazides, hydroxylamines, thiols, alcohols and phenols, hydroperoxides, boronic acids, carbonyl compounds (especially aldehydes, ketones, carboxylic acids, esters, carbamates, ureas), thio-carbonyl compounds and di-(thio) carbonyl compounds, sulfoxides, sulfones, sulfinic acids, sulfonic acids, olefines and alkynes.

12. The method according to claim 1 or 11, wherein the set of ligand precursors comprises at least 10 different ligand precursors.

13. The method according to any of the claims 1 to 12, wherein the precursors have two different functional groups capable of covalently binding two different sets of reactants.

14. The method according to claim 13, wherein the two different functional groups are selected independently from the group consisting of amino groups, hydrazines and hydrazides, thiosemicarbazides, hydroxylamines, thiol groups, alcohol and phenol groups, borates derivatives, carboxy groups and derivatives thereof, thio-carboxy and di-(thio) carboxy groups and derivatives thereof, sulfo groups, sulfonato groups and sulfamoyl groups, olefines, alkynes, halides (especially arylic or benzylic), isocyanate and isothiocyanate groups and nitriles groups.

15. The method according to any of the claims 1, 2 or 4 to 14, wherein the set of reactants is added as a solution comprising a mixture of all reactants.

16. The method according to any of the claims 1 to 15, wherein the ligand precursors or reactants are fragments of known binders to the target.

17. The method according any of the claims 4 to 16, wherein before step (f) the unbound reactants are removed from the solid phase.

18. The method according to claim 4 or 17, wherein the ligand is identified after removal from the solid phase.

19. The method according to claim 18, wherein the ligand is removed and analyzed by MALDI MS.

20. The method according to any of the claims 4 to 19, wherein the ligand is identified by repeating steps (a) to (e) of claim 1 or steps (a) to (g) of claim 2 by subsequently reducing the number of reactants.

21. The method according to any of the claims 1 to 20, wherein the molecular weight of each ligand is less than 1000 g/mol.

22. The method according to any of the claims 1 to 16, wherein the target is a protein.

23. The method according to any of the claims 1 to 22, wherein the ligand precursors are immobilized on a solid phase comprising an organic layer attached to a metal film on a solid support.

24. The method according to claim 23, wherein the organic layer is a self assembled monolayer comprising anchor molecules.

25. The method according to claim 24, wherein the monolayer comprises additionally dilution molecules.

26. The method according to claim 24 or 25, wherein the ligand is removed by photo cleavage of an anchor molecule.

27. The method according to claim 25 or 26, wherein steps (a) to (e) of claim 1 or steps (a) to (g) of claim 2 are repeated using different dilutions.

28. The method according to any of the claims 1 to 27 wherein the ligand precursors are position addressable.

29. The method according to claim 28, wherein the ligand precursor is identifiable by its coordinates on the solid phase.

30. The method according any of the claims 23 to 29, wherein the metal film is gold.

31. The method according to any of the claims 1 to 30, wherein the binding value corresponds to an SPR signal.
